# EUROPEAN PATENT APPLICATION

(11) **EP 1 731 898 A1**
(43) Date of publication of application: **13.12.2006**
(21) Application number: 05719873.1
(22) Date of filing: 03.03.2005
(51) Int. Cl.: G01N 21/78, C07D 311/82, C09B 11/28

(54) **FLUORESCENT PROBES**

(30) Priority: 04.03.2004 JP 2004060080
(71) Applicant: Nagano, Tetsuo, Suginami-ku, Tokyo 167-0032 (JP); DAIICHI PURE CHEMICALS CO., LTD., Chuo-ku Tokyo 103-0027 (JP)
(72) Inventor: NAGANO, Tetsuo, Tokyo 1670032 (JP); WADA, Yoko, 42, Tokyo University Shirokane dorm., Tokyo 1080072 (JP); URANO, Yasuteru, Kawasaki-shi, Kanagawa 2130013 (JP)
(74) Representative: Sternagel, Fleischer, Godemeyer & Partner
(86) International application number: PCT/JP2005/003569
(87) International publication number: WO 2005/085811

(57) **Abstract**

A fluorescent probe which is represented by the following formula (wherein R¹ and R² represent hydrogen atom, or a substituent for trapping proton, a metal ion, or an active oxygen species; R³ represents a monovalent substituent other than hydrogen atom, carboxy group, or sulfo group; R⁴ and R⁵ represent hydrogen atom, a halogen atom, or an alkyl group; R⁶ to R⁹ represent an alkyl group; R¹⁰ and R¹¹ represent hydrogen atom, a halogen atom, or an alkyl group; M⁻ represents a counter ion; and the combination of R¹, R², and R³ (1) imparts a substantially high electron density to the benzene ring to which they bond so that the compound can be substantially non-fluorescent before trapping proton, a metal ion, or an active oxygen species, and (2) substantially reduces electron density of the benzene ring to which they bond so that the compound after trapping proton, a metal ion, or an active oxygen species can be highly fluorescent after the trapping.

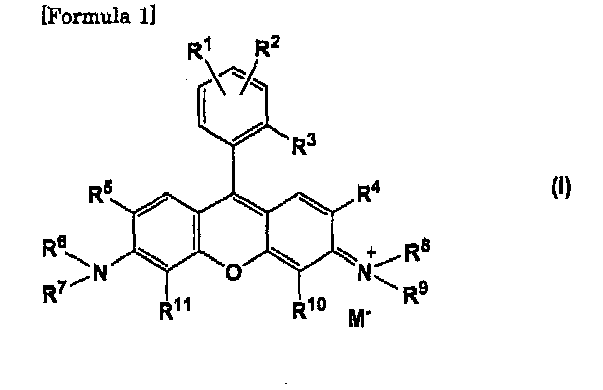

## Description

### Technical Field

The present invention relates to a fluorescent probe. More specifically, the present invention relates to a fluorescent probe that traps proton, metal ion, or active oxygen species to emit fluorescence.

### Background Art

Rhodamines are fluorescent dyes known for many years like fluoresceins. Since both exhibit a high fluorescence quantum yield in water, they have been widely applied in the field of biology as fluorescent tags. Live cell imaging techniques using a fluorescent probe have come to be frequently used in recent years, and both types of dyes are also widely used as basic structures of fluorescent probes which play a significant role in said techniques. In particular, fluoresceins are most widely used basic structures, and for example, fluo-3 which is a calcium fluorescent probe (R.Y Tsien, et al., J. Biol. Chem., 264, 8171, 1989), sodium green which is a sodium fluorescent probe (U.S. Patent No. 5,405,975), DAFs which are NO fluorescent probes (Japanese Patent Unexamined Publication (Kokai) No. 10-226688), HPF and APF which are active oxygen fluorescent probes (International Patent Publication WO01/064664), and the like are well known.

Since the fluorescence imaging techniques are those enabling measurement of cells in a living state, they are becoming essential techniques for elucidation of signal transduction mechanisms and the like. For such a purpose, if only a single kind of molecule as a measuring object is to be visualized, it is sufficient to develop a fluorescent probe having a fluorescein structure. However, various kinds of events occurring in cells are complicated, and it is very often required to simultaneously visualize two or more kinds of measuring object molecules. In that case, it is essential to develop two or more kinds of fluorescent probes which can function in different wavelength regions. For this purpose, a combination of a fluorescein and a rhodamine is an ideal combination, because both are long wavelength excitation dyes causing little cell damages, each wavelength region is sufficiently separated, i.e., excitation/emission wavelengths of the former are 492/515 nm, and those of the latter are 550/570 nm, both are bright dyes having fluorescence quantum yields near 1 and extremely large molar extinction coefficients almost equal to 100,000 and the like.

Although some kinds of fluorescent probes having a rhodamine structure have already been developed so far, their variety are still very limited, and therefore objects of visualization are limited to a very small kinds of molecules. Moreover, rhodamines suffer from very low purification efficiency by colum chromatography, and the molecules are hard to be handled from a synthetic viewpoint. Therefore, even synthesis of derivatives introduced only one substituent is extremely difficult. This fact is also a reason why development of fluorescent probes having a rhodamine structure has been discouraged.

The inventors of the present invention previously found that fluorescence quantum yields of fluoresceine were successfully controlled by intramolecular photoinduced electron transfer, and they constructed methods for logically designing fluorescein fluorescent probes on the basis of this finding. They also succeeded in modification of the molecular structures of fluoresceins, and based on the result, they successfully established a method for precisely designing fluorescent probes having a fluorescein structure that can use a wide variety of molecular species as objects of visualization (PCT/JP03/08585). Further, they found that, in fluoresceins, carboxy group conventionally considered to be indispensable for intense fluorescent property was replaceable with other substituents, and based on this finding, they successfully synthesized various novel fluorescein derivatives (PCT/JP/08585). Furthermore, they also contrived an innovatively novel method in which these novel chemical structures were applied, and succeeded in development of probes based on this finding (PCT/JP03/08585, Japanese Patent Application No. 2003-314041). However, such researches have not been conducted on fluorescent probes having a rhodamine structure so far, and development of fluorescent probes having a rhodamine structure is made on a trial and error basis even at present.

### Disclosure of the Invention

### Object to be achieved by the invention

An object of the present invention is to provide a novel fluorescent probe having a rhodamine structure. Another object of the present invention is to provide a means for designing a novel fluorescent probe having a rhodamine structure.

### Means for achieving the object

The inventors of the present invention applied the aforementioned precise design method, that was developed for fluoresceins, to the rhodamine structure, and as a results, they succeeded in development of novel rhodamines. These rhodamine derivatives have remarkable characteristics that they have a strong fluorescent property comparable to that of known rhodamines, and moreover, they can be synthesized in a very high yield. They also found that, by using novel parameters different from those applied for fluoresceins as parameters for designing fluorescent probes, the quantum yield of the rhodamine derivatives was successfully controlled, as desired, on the basis of the photoinduced electron transfer (PET) as a basic principle in the same manner as fluoresceins. On the basis of these findings, it became possible to convert a basic skeleton of a fluorescent probe having a fluorescein structure to a rhodamine structure, and thereby control the excitation and emission wavelengths as desired.

The present invention thus provides a fluorescent probe which is represented by the following formula (I): 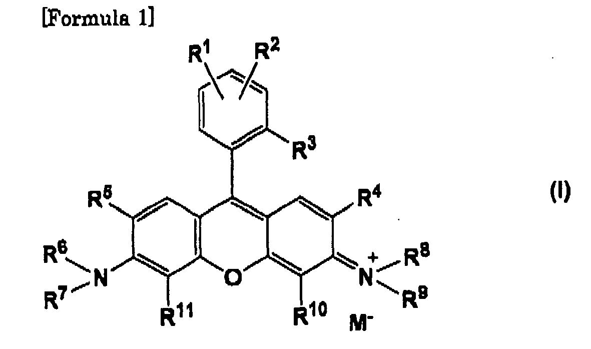 wherein R¹ and R² each independently represent hydrogen atom, or a substituent for trapping proton, a metal ion, or an active oxygen species, provided that R¹ and R² do not simultaneously represent hydrogen atom, or R¹ and R² may combine to each other to form a ring structure for trapping proton, a metal ion, or an active oxygen species; R³ represents a monovalent substituent other than hydrogen atom, carboxy group, or sulfo group; R⁴ and R⁵ each independently represent hydrogen atom, a halogen atom, or an alkyl group which may have or a substituent; R⁶, R⁷, R⁸, and R⁹ each independently represent an alkyl group which may have a substituent; R¹⁰ and R¹¹ each independently represent hydrogen atom, a halogen atom, or an alkyl group which may have a substituent; in one or more combinations selected from the group consisting of combinations of R⁴ and R⁸, R⁹ and R¹⁰, R⁵ and R⁸, and R⁷ and R¹¹, two of the groups included in each combination (wherein these groups are alkyl groups which may have a substituent) may combine to each other to form a 5- or 6-membered ring; and M-represents a counter ion; provided that combination of R¹, R², and R³
(1) imparts a substantially high electron density to the benzene ring to which they bond so that the compound represented by the formula (I) can be substantially non-fluorescent before trapping proton, a metal ion, or an active oxygen species, and
(2) substantially reduces electron density of the benzene ring to which they bond so that the compound derived from the compound represented by the formula (I) after trapping proton, a metal ion, or an active oxygen species can be highly fluorescent after the trapping.

According to preferred embodiments of the aforementioned invention, there are provided the aforementioned fluorescent probe, wherein the benzene ring on which R¹, R², and R³ substitute has an oxidation potential less than 1.20 V before trapping proton, a metal ion, or an active oxygen species, and an oxidation potential not less than 1.40 V after trapping proton, a metal ion, or an active oxygen species; the aforementioned fluorescent probe, wherein R³ is a lower alkyl group, or a lower alkoxy group; the aforementioned fluorescent probe, wherein the metal ion is an alkali metal ion, calcium ion, magnesium ion, or zinc ion; and the aforementioned fluorescent probe,
wherein the active oxygen species is selected from the group consisting of nitric oxide, hydroxyl radical, singlet oxygen, and superoxide.

According to more preferred embodiments, there are provided the aforementioned fluorescent probe, which is a fluorescent probe for measuring zinc ion or nitric oxide, and wherein one or both of R¹ and R² are groups represented by the following formula (A): 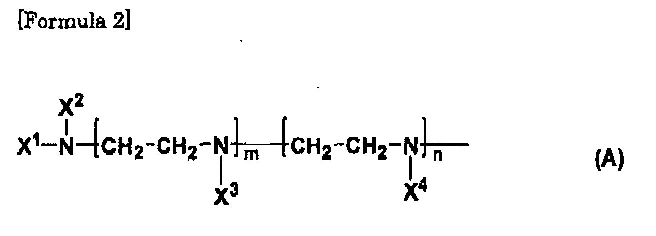 wherein X¹, X², X³, and X⁴ each independently represent hydrogen atom, an alkyl group, 2-pyridylmethyl group, or a protective group of amino group, and m and n each independently represent 0 or 1; and the aforementioned fluorescent probe, which is a fluorescent probe for measuring singlet oxygen, and wherein R¹ and R² combine to each other to represent a ring structure represented by the following formula (B); 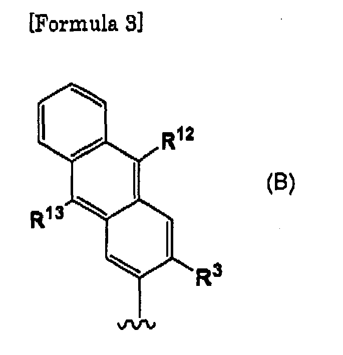 wherein R¹² and R¹³ each independently represent a C₁₋₄ alkyl group, or an aryl group.

From another aspect, the present invention provides a method for designing a fluorescent probe represented by the formula (I) mentioned above, wherein R¹ and R² each independently represent hydrogen atom, or a substituent for trapping proton, a metal ion, or an active oxygen species, provided that R¹ and R² do not simultaneously represent hydrogen atom, or R¹ and R² may combine to each other to form a ring structure for trapping proton, a metal ion, or an active oxygen species; R⁸ represents a monovalent substituent other than hydrogen atom, carboxy group, or sulfo group; R⁴ and R⁵ each independently represent hydrogen atom, a halogen atom, or an alkyl group which may have or a substituent; R⁶, R⁷, R⁸, and R⁹ each independently represent an alkyl group which may have a substituent; R¹⁰ and R¹¹ each independently represent hydrogen atom, a halogen atom, or an alkyl group which may have a substituent; in one or more combinations selected from the group consisting of combinations of R⁴ and R⁵, R⁶ and R¹⁰, R⁵ and R⁶, and R⁷ and R¹¹, two of the groups included in each combination (wherein these groups are alkyl groups which may have a substituent) may combine to each other to form a 5- or 6-membered ring; and M' represents a counter ion, which comprises the step of selecting R¹, R², and R³ as a combination that;
(1) imparts a substantially high electron density to the benzene ring to which they bond so that the compound represented by the formula Q) can be substantially non-fluorescent before trapping proton, a metal ion, or an active oxygen species, and
(2) substantially reduces electron density of the benzene ring to which they bond so that the compound derived from the compound represented by the formula (I) after trapping proton, a metal ion, or an active oxygen species can be highly fluorescent after the trapping.

### Effect of the invention

A fluorescent probe having a novel rhodamine structure, and a means for designing a novel fluorescent probe having a rhodamine structure are provided by the present invention.

### Brief Description of the Drawings

[Fig.1] Fig. 1 shows relationship between the fluorescence quantum yields of Compounds 1 to 8 and oxidation potentials of PET donor moieties.
[Fig.2] Fig. 2 shows relationship between the fluorescence intensity of Compound 1 and pH change.

### Best Mode of Carrying Out the Invention

The fluorescent probe provided by the present invention, which is represented by the formula (I), is used as a fluorescent probe for measuring proton, a metal ion, or an active oxygen species (in the specification, these are also referred to as "measuring object"). Examples of the metal ion include as alkali metal ions such as sodium ion and lithium ion, alkaline earth metal ions such as calcium ion, magnesium ion, zinc ion, and the like. Examples of the active oxygen species include nitric oxide, hydroxy radical, singlet oxygen, superoxide, and the like. However, the measuring object is not limited to these examples.

The fluorescent probe of the present invention is characterized in that the probe is obtained by replacing the carboxy group of the 2-carboxyphenyl group, that binds to the 9-position of the xanthene ring of fluorescent probes comprising rhodamine as a basic structure conventionally proposed for measurement of various measuring objects, with a monovalent substituent other than hydrogen atom or sulfo group (in the formula (I), this substituent is represented by R⁸). As fluorescent dyes comprising rhodamine as a basic structure, such compounds in which one or more rings are further added to the xanthene ring are known. For example, compounds having seven rings so that the molecules contain a julolidine structure, such as X-Rhodamine (2-carboxyphenyl group binding to the 9-position of the xanthene ring moiety), Texas Red (carboxy group of 2-carboxyphenyl group bonding to the 9-position of the xanthene ring is replaced with sulfo group), and the like are known, and as the fluorescent probes having seven rings, Calcium Crimson, which is a calcium fluorescent probe, AM and the like are known (as for the substances mentioned above, see, the catalog of Molecular Probes, Inc. (Handbook of Fluorescent Probes and Research Chemicals, Ninth edition), pages 57-65 and 788-790). The fluorescent probe of the present invention include the compounds in which one or more rings are added to the xanthene ring, including the compounds having seven rings.

For example, the compounds of the present invention having seven rings can be prepared by synthesizing a xanthone from formaldehyde and 8-hydroxyjulolidine according to the method described in J. Prakt. Chem., 54, 223 (1896); Dyes and Pigments, 42, 71 (1999), or the like and binding the benzene ring moiety according to the method described in the examples.

On the benzene ring binding to the 9-position of the xanthene ring, two substituents are present either one of which or a combination thereof participates in trapping of a measuring object (in the formula (I), these substituents are represented by R¹ and R², either one of which may represent hydrogen atom). As R¹ and R² in the compound represented by the formula (I), substituents for trapping a measuring object, which have conventionally been used for fluorescent probes for measuring proton, a metal ion, or an active oxygen species, can be used. R¹ and R² on the benzene ring may combine to each other to form a ring structure and thereby form a substituent for trapping proton, a metal ion, or an active oxygen species. For example, as a combination of R¹ and R² on the benzene ring, groups shown below can be used. However, the combination is not limited to these examples (2-substituted phenyl groups which bind to the 9-position of the xanthene ring or rings condensed with said phenyl group are shown).

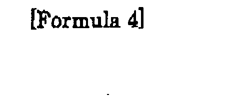

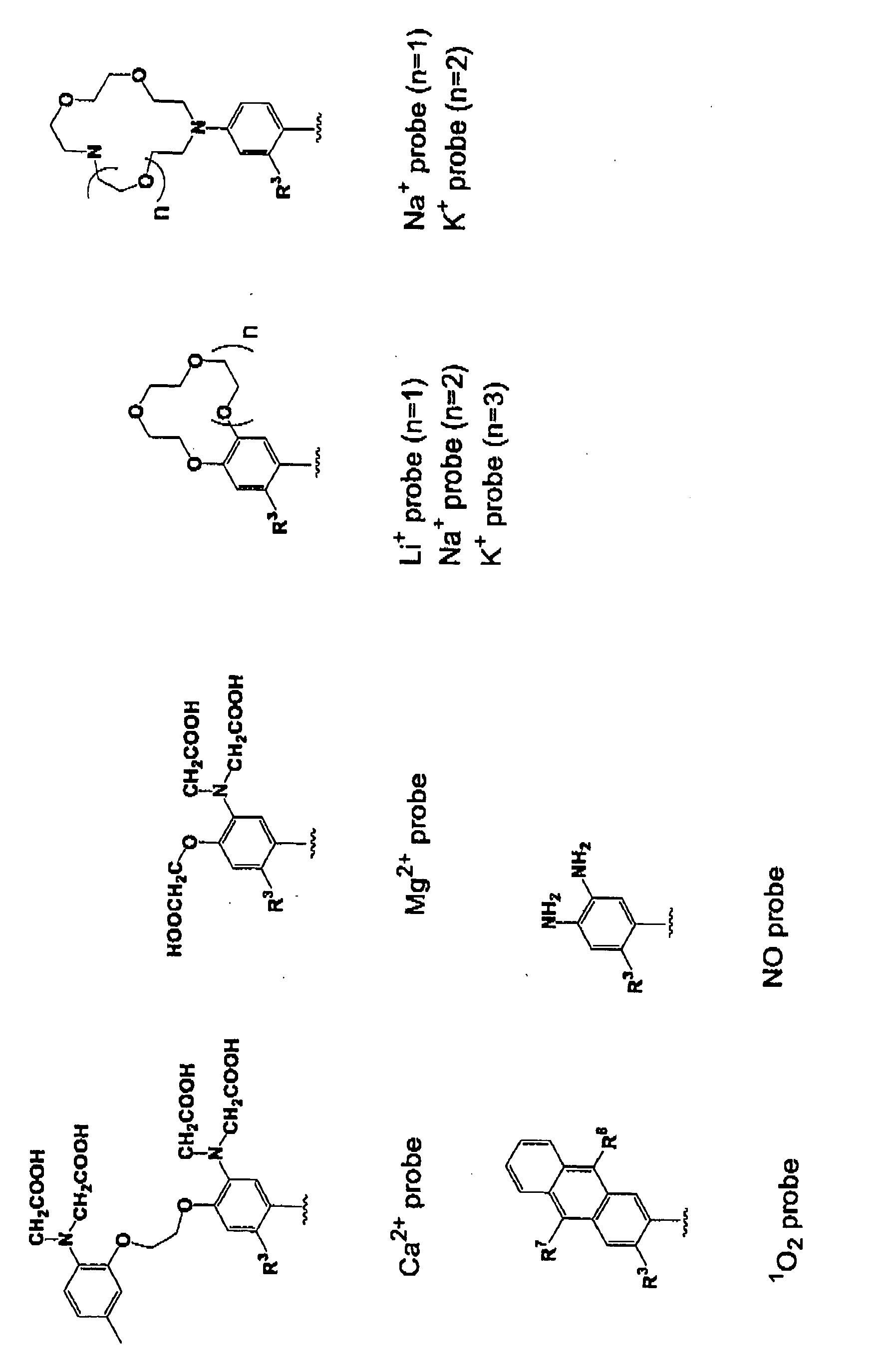

The substituting positions of R¹ and R² on the benzene ring are not particularly limited. On the benzene ring to which R¹, R², and R³ bind, any substituent other than these substituents may be present. Various kinds of substituents for trapping a measuring object have been proposed, and a person skilled in the art can suitably select a substituent depending on the type of a measuring object. For example, Japanese Patent Unexamined Publication No. 10-226688, WO99/51586, Japanese Patent Unexamined Publication No. 2000-239272, WO01/62755, and the like can be referred to. Also usable are substituents for trapping measuring objects described in the catalog of Molecular Probes Inc. (Handbook of Fluorescent Probes and Research Chemicals, Sixth edition), Chapter 20 (calcium ion, magnesium ion, zinc ion, and the other metal ions), Chapter 21 (pH indicator), and Chapter 22 (sodium ion, potassium ion, chloride ion, and the other inorganic ions), However, substituents for trapping measuring objects are not limited to those described in the aforementioned publications.

In the specification, the term "trapping" should be construed in its broadest sense which includes trapping of a metal ion without substantially causing chemical transformation of R¹ and/or R² such as chelating, as well as trapping causing change of the chemical structures of R¹ and/or R² by chemical reaction with a measuring object, and should not be construed in any limitative sense.

For example, for a fluorescent probe for measuring zinc ion or nitric oxide, either one or both of R¹ and R² are preferably groups represented by the following formula (A): 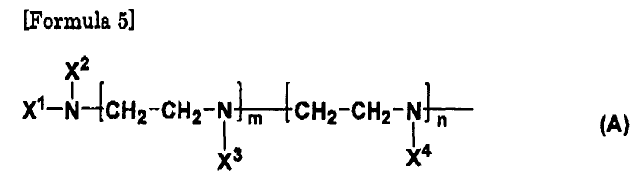 wherein X¹, X², X³, and X⁴ each independently represents hydrogen atom, an alkyl group, 2-pyridylmethyl group, or a protective group of amino group, and m and n each independently represents 0 or 1.

For a fluorescent probe for measuring nitric oxide, both of R¹ and R² independently represent a group represented by the aforementioned formula (A),
wherein m and n represent 0, and R¹ and R² substitute on the benzene ring at adjacent positions. For a fluorescent probe for measuring zinc ion, it is preferred that one of R¹ and R² is a group represented by the aforementioned formula (A), and the other is hydrogen atom, wherein X¹, X², X³, and X⁴ are preferably 2-pyridylmethyl groups, and more preferably X¹ and X² are 2-pyridylmethyl groups. It is preferred that m is 0, n is 1, and X⁴ is hydrogen atom, and in this case, both of X¹ and X² are preferably 2-pyridylmsthyl groups.

For a fluorescent probe for measuring singlet oxygen, R¹ and R² preferably combine to each other to represent a ring structure represented by the following formula (B): 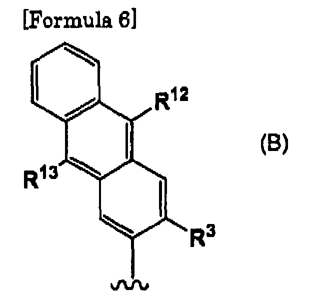 wherein R¹² and R¹³ each independently represents a C₁₋₄ alkyl group, or an aryl group. It is preferred that R¹² and R¹³ each independently represent a phenyl group which may have a substituent, and it is more preferred that both of R⁷ and R⁸ represent phenyl group. The aforementioned formula (B) represents a group which binds at the 9-position of the xanthene ring, and one or more substituents may be present at any substitutable positions on the ring of the aforementioned formula (R).

In the specification, "an alkyl group" or an alkyl moiety of a substituent containing the alkyl moiety (for example, an alkylcarbonyl group, an alkylcarbonyloxymethyl group and the like) means, for example, a linear, branched, or cyclic alkyl group, or an alkyl group consisting of a combination thereof, having 1 to 12 carbon atoms, preferably 1 to 6 carbon atoms, more preferably 1 to 4 carbon atoms. More specifically, a lower alkyl group (an alkyl group having 1 to 6 carbon atoms) is preferred as the alkyl group. Examples of the lower alkyl group include methyl group, ethyl group, n-propyl group, isopropyl group, cyclopropyl group, n-butyl group, sec-butyl group, isobutyl group, tert-butyl group, cyclopropylmethyl group, n-pentyl group, n-haxyl group, and the like. The "halogen atom" referred to in the specification may be any one of fluorine atom, chlorine atom, bromine atom, and iodine atom, preferably, fluorine atom, chlorine atom, or bromine atom.

As R³, a lower alkyl group or a lower alkoxy group is preferred. Particularly preferred is methyl group or methoxy group. As the halogen atom represented by R⁴ and R⁵, chlorine atom or fluorine atom is preferred. It is preferred that R⁴ and R⁵ each independently represent hydrogen atom, chlorine atom, or fluorine atom. As the alkyl group represented by R⁶, R⁷, R⁸, and R⁹, a lower alkyl group such as methyl group is preferred. R¹⁰ and R¹¹ preferably represent hydrogen atom. When groups included in each of one or more combinations selected from the group consisting of combinations of R⁴ and R⁸, R⁹ and R¹⁰, R⁵ and R⁶, and R⁷ and R¹¹, two of the groups included in each combination (wherein these groups are alkyl groups which may have a substituent) bind to each other to form a 5- or 6-membered ring, the ring to be formed is preferably a 6-membered ring containing nitrogen atom binding to the 3'- or 6'-carbon atom in which the groups are bound to each other via trimethylene chain. In the group represented by the formula (A), type of the protective group of amino group is not particularly limited. For example, p-nitrobenzenesulfonyl group, trifluoroacetyl group, trialkylsilyl group, and the like can be suitably used. As for the protective group of amino group, for example, "Protective Groups in Organic Synthesis," (T.W. Greene, John Wiley & Sons, Inc. (1981)), and the like can be referred to.

In the compound represented by the aforementioned formula (I), M- represents a counter ion, and means counter ions in a number sufficient for neutralizing the charge of the molecule. Type of the counter ion is not particularly limited, and examples include, for example, chlorine ion, sulfate ion, nitrate ion, organic acid anions such as methanesulfonate anion, p-tolueneeulfonate anion, oxalate anion, citrate anion and tartrate anion, and the like. Carboxy anions of amino acids such as glycine may also be used.

In the fluorescent probe of the present invention, the combination of R¹, R², and R³ is selected as a combination that (1) imparts a substantially high electron density to the benzene ring to which they bond so that the compound represented by the formula (I) can be substantially non-fluorescent, and (2) substantially reduces electron density of the benzene ring to which they bond so that the compound derived from the compound represented by the formula (I) after trapping of a measuring object can be substantially highly fluorescent after the trapping.

Information of the electron density of the benzene ring to which R¹, R², and R³ bind can be easily obtained, for example, by calculating oxidation potential of the benzene ring using a quantum chemical technique. A reduction of the oxidation potential of the benzene ring means an increase of the electron density of the benzene ring, which corresponds to an elevation of HOMO orbital energy. For example, HOMO energy of the benzene ring moiety can be determined according to the density functional theory (B3LYP/6-31G(d)). As R¹ and R², substituents should be selected which change the oxidation potential after trapping of a measuring object. All the oxidation potentials described in the specification are indicated as values obtained by using a saturated calomel electrode (SCE) as a reference electrode, of which standard is different by about 0.24 V from the value where a silver nitrate electrode (Ag/Ag⁺) is used as a reference electrode.

For example, as for the compound represented by the general formula (I), a compound wherein the oxidation potential of the benzene ring is 1.00 V or lower may be substantially non-fluorescent, whilst a compound wherein the oxidation potential of the benzene ring is 1.40 V or higher may be substantially strongly fluorescent. When the combination of R¹, R², and R³ is selected by using the oxidation potential of the benzene ring as a criterion, a fluorescent probe having an excellent fluorescent property can be obtained by selecting the combination as a combination that (1) imparts a substantially high electron density to the benzene ring in the compound before trapping a measuring object, and (2) substantially reduces electron density of the benzene ring after trapping a measuring object.

Although it is not intended to be bound by any specific theory, the above mentioned findings can be explained on the basis of PET (Photoinduced Electron Transfer). PET is one of methods for fluorescence quenching, wherein electron transfer from neighboring electron donating moiety (PET donor) occurs faster than returning of the singlet-excited fluorescent group generated by irradiation of excitation light to the ground state with fluorescence emission, and thereby fluorescence quenching is induced. When the compound represented by the formula (I) is divided for consideration into the xanthene ring moiety which acts as a fluorescent group and the benzene ring moiety which is a moiety for quenching fluorescence (PET donor), if the oxidation potential of the benzene ring is low (i.e., electron density and HOMO energy are high), the fluorescence derived from the xanthene ring will be quenched through the PET.

As fluorescent probes, compounds are required to have a property that they are substantially non-fluorescent before trapping of a measuring object and change into a substantially strongly fluorescent substance after trapping of a measuring object. Therefore, a probe showing a significant change in fluorescence intensity can be chosen as a preferred probe. For example, a probe can be designed so that its fluorescence is quenched through PET before trapping of a measuring object and substantially no PET is induced after trapping of a measuring object. When a fluorescent probe introduced with a novel substituent as R¹, R², and/or R³ is designed by using the oxidation potential of the benzene ring moiety as a criterion, correlation between the oxidation potential of the benzene ring after the introduction of the functional group and weakening of fluorescence may be predicted from the knowledge available at present. Nevertheless, the correlation between the oxidation potential and the fluorescence intensity is preferably determined by the method specifically described in the examples of the specification.

Further, for example, when a fluorescent probe for measuring nitric oxide is designed, electron density of the adjacent amino groups represented by R¹ and R² (either one of the amino groups may be substituted, for example, with an alkyl group) can be increased to increase the reactivity between nitric oxide and the amino groups and thus increase sensitivity of the fluorescent probe. If an electron donating group such as an alkyl group and an alkoxy group is used as R³ in the fluorescent probe of the present invention, electron density of the benzene ring is increased, and as a result, the substantially non-fluorescent property before trapping of nitric oxide can be maintained, and the electron densities of the amino groups are also increased to improve reactivity with nitric oxide. Similarly, in a fluorescent probe for measuring singlet oxygen, by increasing electron density of the reactive group represented by the aforementioned formula (B), reactivity with singlet oxygen can be increased, and it becomes possible to maintain the substantially non-fluorescent property of the fluorescent probe before trapping of singlet oxygen.

The term "measurement" used in this present specification should be construed in its broadest sense, including determinations, tests, and detections performed for the purpose of quantification, qualification, diagnosis or the like. The method for measuring a measuring object using the fluorescent probe of the present invention generally comprises (a) the step of reacting a compound represented by the aforementioned formula (I) with a measuring object; and (b) the step of measuring fluorescence of a compound generated in the aforementioned step (a). For example, the fluorescent probe of the present invention or a salt thereof may be dissolved in an aqueous medium such as physiological saline or a buffer, or in a mixture of an aqueous medium and a water-miscible solvent such as ethanol, acetone, ethylene glycol, dimethyl sulfoxide, and dimethylformamide, the resultant solution may be added to a suitable buffer containing cells or tissues, and then the fluorescence spectra may be measured.

Fluorescence of the compounds after trapping a measuring object can be measured by an ordinary method. For example, a method of measuring fluorescence spectra *in vitro,* a method of measuring fluorescence spectra *in vivo* by using a bioimaging technique, and the like can be employed. For example, when a quantitative measurement is conducted, it is desirable that a calibration curve is prepared in advance in a conventional manner.

The fluorescent probe of the present invention may be used as a composition by mixing with additives generally used for preparation of measurement reagents, if necessary. For example, as additives for use of regents under a physiological condition, additives such as dissolving aids, pH adjusters, buffers, and isotonic agents can be used, and amounts of these additives can suitably be chosen by those skilled in the art. The compositions may be provided as those in appropriate forms, for example, powdery mixture, lyophilized product, granule, tablet, solution, and the like.

The compound provided from another aspect of the present invention is represented by the following formula (II): 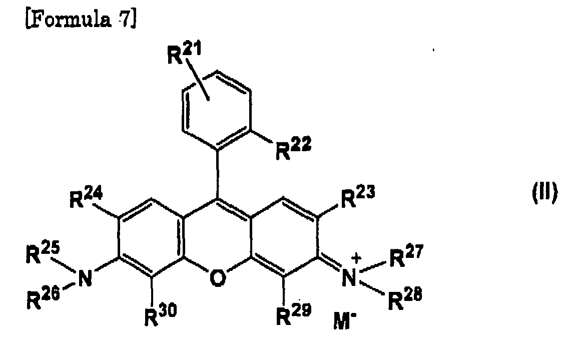 wherein R²¹ represents hydrogen atom, an alkyl group, or an alkoxy group; R²² represents an alkyl group, or an alkoxy group; R²³ and R²⁴ each independently represents hydrogen atom, a halogen atom, or an alkyl group which may have a substituent; R²⁵, R²⁶, R²⁷, and R²⁸ each independently represent an alkyl group which may have a substituent; R²⁹ and R³⁰ each independently represent hydrogen atom, a halogen atom, or an alkyl group which may have a substituent; in one or more combinations selected from the group consisting of combinations of R²³ and R²⁷, R²⁸ and R²⁹, R²⁴ and R²⁵, and R²⁶ and R³⁰, two of the groups included in each combination (wherein these groups are alkyl groups which may have a substituent) may combine to each other to form a 5- or 6-membered ring; and M⁻ represents a counter ion. As R²¹, hydrogen atom, an alkyl group having 1 to 4 carbon atoms, or an alkoxy group having 1 to 4 carbon atoms is preferred, and hydrogen atom, methyl group, or methoxy group is preferred. As R²², an alkyl group having 1 to 4 carbon atoms, or an alkoxy group having 1 to 4 carbon atoms is preferred, and methyl group, or methoxy group is more preferred. R²⁸ and R²⁴ preferably represent hydrogen atom. R²⁵, R²⁶, R²⁷, and R²⁸ preferably represent methyl group. R²⁹ and R³⁰ preferably represent hydrogen atom. This compound can be used for a donor or acceptor in measurement of a measuring object using fluorescence resonance energy transfer (FRET), and the like.

The compound represented by the aforementioned formula (II) can be easily synthesized by the method specifically described in the examples of the specification. Conventionally, a synthetic method based on a condensation reaction using a monosubstituted phthalic anhydride is common as the method for preparing rhodamines having a substituent on the benzene ring moiety. However, a mixture of two kinds of isomers is obtained by employing the above method, and it is often difficult to separate those isomers. Moreover, conventional rhodamine derivatives exhibit strong adsorption to silica gel carriers and thus give very poor purification efficiency Therefore, a yield very often fails to reach even 10%. On the other hand, the rhodamines of the present invention represented by the aforementioned formula (II) can be produced by one step of a C-C bond formation reaction using a lithium reagent, and in addition, that method solely provides the target molecule having a single kind of basic structure and avoids necessity of isomer separation. Moreover, the compounds have a feature of weak adsorption to silica gel carriers and thus achieves high purification efficiency. Generally, it is possible to prepare the target substances in a yield over 90%.

### Examples

The present invention will be explained more specifically by referring to the following examples. However, the scope of the present invention is not limited to these examples.

### Example 1: Preparation of compounds

The following compounds were prepared. These compounds were designed so that the compound appointed with a higher compound number had a lower oxidation potential of the benzene ring that binds at the 9-position of the xanthene ring (i.e., so as to have a higher electron density, in other words, a higher HOMO orbital energy). Preparation scheme of Compound 1 is shown below.

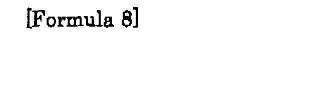

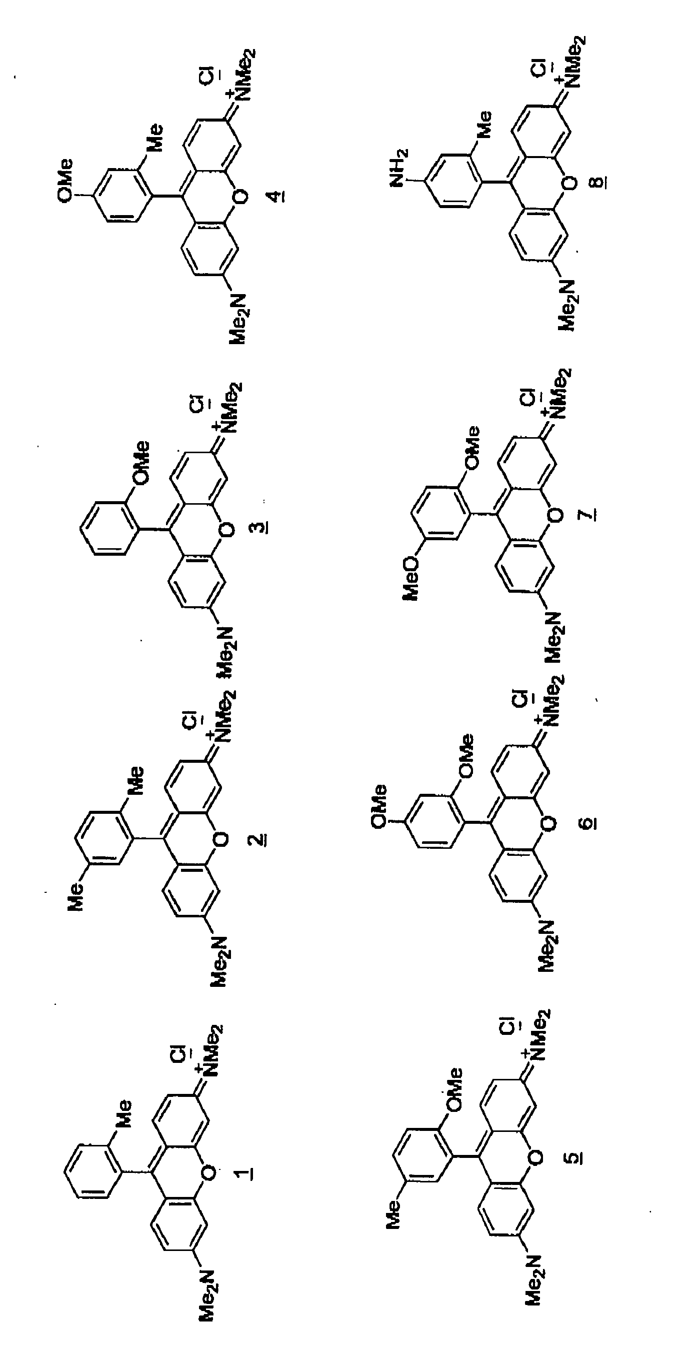

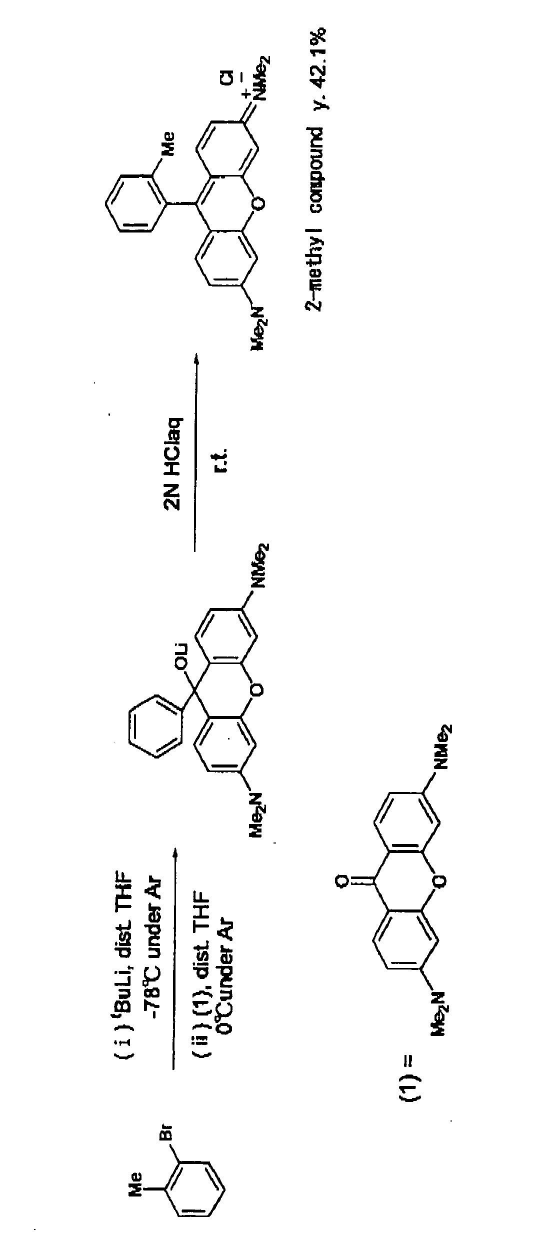

3,6-Bisdimethylaminoxanthone was synthesized by referring to J. Prakt. Chem., 54, 223 (1896) and Dyes and Pigments, 42, 71 (1999). 2-Bromotoluene (92.4 mg, 0.54 mmol) dissolved in tetrahydrofuran (THF, 1 ml) was put into a sufficiently dried vessel under an argon atmosphere, and cooled to -78°C on a dry ice/acetone bath. This solution was added with tert-butyllithiuna (1.54 mol, in 1 ml of n-pentane), and 3,6-bisdimethylaminoxanthone (50 mg, 0.18 mmol) dissolved in THF (2 ml), and the mixture was stirred for 30 minutes. The reaction mixture was added with 2 N aqueous HCl (10 ml), and the mixture was stirred for 30 minutes, concentrated, and then extracted with methylene chloride. The organic layer was concentrated, and the resulting residue was purified by silica gel column chromatography (methylene chloride/methanol = 19/1) to obtain purple solid of Compound 1 (30 mg, yield: 42.1%).
¹H-NMR (300MHz, CDCl₃) δ ppm 2.04 (3H, s), 3.39 (12H, s), 6.98 (2H, s), 7.01 (2H, d, J=2.4Hz), 7.16-7.26 (3H, m), 7.41-7.46 (2H, m), 7.49-7.52 (1H,m)
MS (FAB) 857 (M-Cl⁻)

Compound 2 was obtained as purple solid (yield: 93.3%) in the same manner as that in the method of (a) mentioned above except that 2-bromo-p-xylene was used instead of 2-bromotoluene.
¹H-NMR (300MHz, CDCl₃) δ ppm 2.04 (3H, s), 2.40 (3H, s), 3.39 (12H, s), 6.95-6.97 (3H, m), 6.99 (2H, dd, J=9.3, 2.4Hz), 7.20 (2H, d, J=9.3Hz), 7.81(2H, m)
MS (FAB) 371 (M-Cl⁻)
Compound 3 was obtained as purple solid (yield: 92.6%) in the same manner as that in the method of (a) mentioned above except that 2-bromo-anisole was used instead of 2-bromotoluene.
¹H-NMR (300MHz, CDCl₃) δ ppm 3.37 (12H, s), 3.73 (3H, s), 6.88 (2H, d, J=2.6Hz), 7.00 (2H, dd, J=9.5, 2.6Hz), 7.14-7.22 (3H, m), 7.29 (2H, d, J=9,5Hz)
MS (FAB) 373 (M-Cl⁻)
Compound 4 was obtained as purple solid (yield: 83.6%) in the same manner as that in the method of (a) mentioned above except that 4-bromo-3-methylanisole was used instead of 2-bromotoluene.
¹H-NMR (300MHz. CDCl₃) δ ppm 2.02 (3H, s), 2.39 (12H, s), 3.92 (3H, s), 6.95-6.97 (4H, m), 6.99 (2H, dd, J=9.5, 2.4Hz), 7.09 (1H, d, J=9.3Hz), 7.24 (2H, d, J=9.5Hz) MS (FAB) 387 (M-Cl⁻)

Compound 5 was obtained as purple solid (yield: 92.7%) in the same manner as that in the method of (a) mentioned above except that 2-bromo-4-methylanisole was used instead of 2-bromotoluene.
¹H-NMR (300MHz, CDCl₃) δ ppm 2.39 (3H, s), 3.38 (12H, s), 3.68 (3H, s), 6.88 (2H, d, J=2.6Hz), 6.97 (1H, d, J=2.6Hz), 7.01 (2H, dd, J=9.5, 2.6Hz), 7.03 (1H, d, J=8.6Hz), 7.31 (2H, d, J=9.5Hz), 7.39 (1H, dd, J=8.6, 2.6Hz)
MS (FAB) 387 (M-Cl⁻)
Compound 6 was obtained as purple solid (yield: 98.0%) in the same manner as that in the method of (a) mentioned above except that 1-bromo-2,4-dimethoxybenzene was used instead of 2-bromotoluens.
¹H-NMR (300MHz, CDCl₃) δ ppm 3.37 (12H, s), 3.71 (3H, s), 3.95 (3H, s), 6.68 (1H, d, J=2.5Hz), 6.72 (1H, dd, J=8.3, 2.5Hz), 6.86 (2H, d, J=2.4Hz), 7.00 (2H, dd, J=9.5, 2.4Hz), 7.11 (1H, d, J=8.3Hz), 7.36 (2H, d, J=9.5Hz)
MS (FAB) 403 (M-Cl⁻)

Compound 7 was obtained as purple solid (yield: 77.6%) in the same manner as that in the method of (a) mentioned above except that 1-bromo-2,5-dimethoxybenzene was used instead of 2-bromotoluene.
¹H-NMR (300MHz, CDCl₃) δ ppm 3.67 (3H, s), 3.82 (12H, s), 3.83 (3H, s), 6.75 (1H, d, J=2.7Hz), 6.90 (2H, d, J=2.4Hz), 7.01 (2H, dd, J=9.5, 2.4Hz), 7.08-7.12 (2H, m), 7.32 (2H, d, J=9.5Hz)
MS (FAB) 403 (M-Cl⁻)
Compound 8 was obtained as purple solid (yield: 58.8 %) in the same manner as that in the method of (a) mentioned above except that 4-bromo-9-methylaniline was used instead of 2-bromotoluene.
¹H-NMR (300MHz, CDCl₃) δ ppm 3.09 (3H, s), 3.37 (12H, s), 6.76 (2H, m), 6.88 (2H, d, J=2.4), 6.90 (1H, m), 6.97 (2H, dd, J=9.5, 2.4Hz), 7.36 (2H, d, J=9.5Hz)
MS (FAB) 872 (M-Cl⁻)

Fluorescent properties and quantum yields (in a buffer of pH 7.4) of the obtained compounds are shown in Table 1 mentioned below.

**[Table 1]**

| Compound No. | Substituent | λ max (nm) | Em(nm) | Φ |
|---|---|---|---|---|
| 1 | 2-Me | 548.8 | 670.4 | 0.644 |
| 2 | 2,5-DiMe | 547.8 | 669.8 | 0.656 |
| 3 | 2-OMe | 553.0 | 577.8 | 0.578 |
| 4 | 2-Me-4-OMe | 550.6 | 569.4 | 0.609 |
| 5 | 2-OMe-6-Me | 552.0 | 576.4 | 0.552 |
| 6 | 2,4-DiOMe | 550.6 | 573.4 | 0.55 |
| 7 | 2,5-DiOme | 554.0 | 581.2 | 0.086 |
| 8 | 2-Me-4-NH₂ | 547.2 | 569.0 | 0.02 |

### Example 2

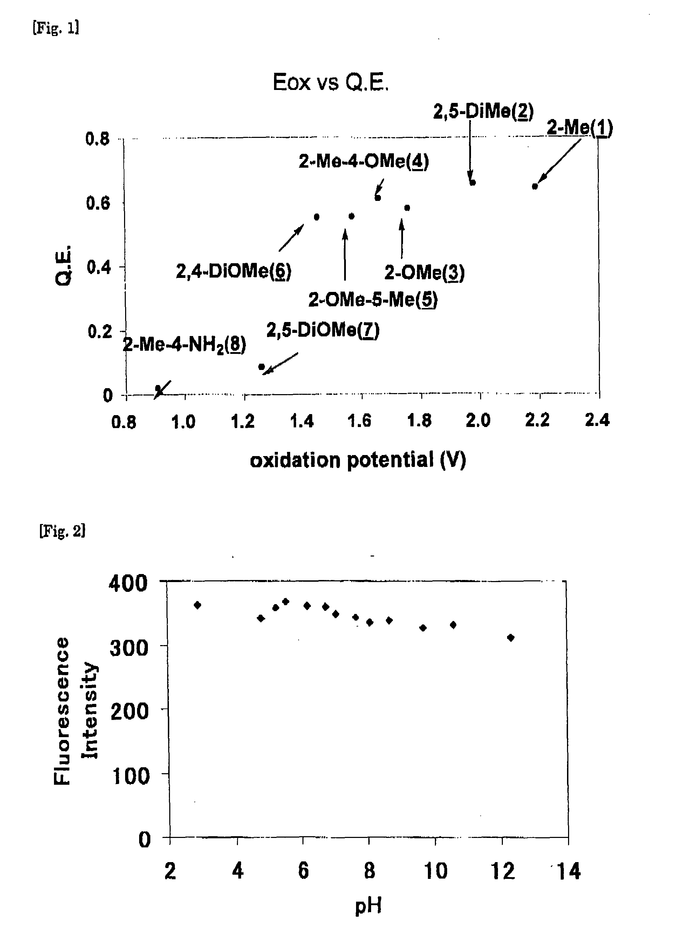

Correlation between the fluorescence quantum yield and the oxidation potential of the benzene ring moiety of each of the compounds synthesized above was studied. The results are shown in Figure 1. As clearly shown by the results shown in the figure, the fluorescence quantum yield of each compound changed depending on the oxidation potential of the benzene ring moiety. From these results, it is clearly understood that the compounds are almost non-fluorescent with an oxidation potential of 1.00 V or lower, whereas with an oxidation potential of 1.40 V or higher, the compounds emitted fluorescence with a quantum yield of about 0.6. Although this oxidation potential-dependent change of the fluorescence quantum yield is substantially similar to that of conventionally known fluorescein derivatives (PCT/JP03/08586), it is considered that the boundary of fluorescence ON/OFF shifts by about 0.1 V to the negative direction. That is, when the same 2,5-climethoxy derivatives of rhodamine and fluorescein are compared, for example, the quantum yield of the rhodamine derivative is about 0.086, whereas the quantum yield of the fluorescein derivative is 0.01 or less. Therefore, it is demonstrated that PET from the benzene ring of the same electron density under a basic condition is more likely to occur in fluoresceins compared with rhodamines. On the basis of this finding, when a probe is prepared by replacing a fluorescein structure with a rhodamine structure, it becomes possible to prepare a probe having equivalent performance by suitably choosing substituents on the benzene ring.

### Example 3

It is known that rhodamines emit stable fluorescence in a broader pH range compared with fluoresceins. It was examined whether this characteristic was maintained in Compounds 1 to 8 mentioned above, which serve as a basic structure of the fluorescent probe of the present invention. Fluorescence intensity of Compound 1 was measured at a fixed concentration in aqueous solutions of various pH values, and plot of the results is shown in Fig. 2. As a result, it was revealed that the compound emitted fluorescence at a substantially constant fluorescence intensity in a broad pH range of 3 to 12.

### Industrial Applicability

A fluorescent probe having a novel rhodamine structure, and a means for designing a novel fluorescent probe having a rhodamine structure are provided by the present invention.

## Claims

1. A fluorescent probe which is represented by the following formula (I): 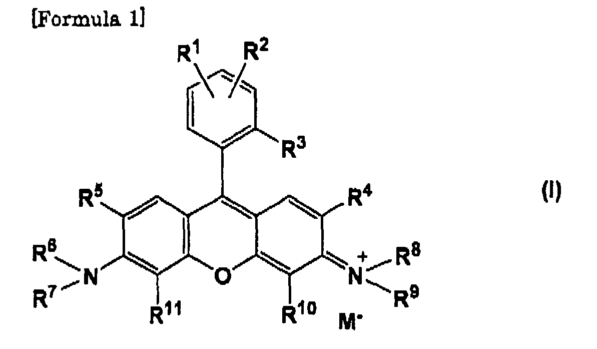 wherein R¹ and R² each independently represent hydrogen atom, or a substituent for trapping proton, a metal ion, or an active oxygen species, provided that R¹ and R² do not simultaneously represent hydrogen atom, or R¹ and R² may combine to each other to form a ring structure for trapping proton, a metal ion, or an active oxygen species; R⁸ represents a monovalent substituent other than hydrogen atom, carboxy group, or sulfo group; R⁴ and R⁵ each independently represent hydrogen atom, a halogen atom, or an alkyl group which may have or a substituent; R⁶, R⁷, R⁸, and R⁹ each independently represent an alkyl group which may have a substituent; R¹⁰ and R¹¹ each independently represent hydrogen atom, a halogen atom, or an alkyl group which may have a substituent; in one or more combinations selected from the group consisting of combinations of R⁴ and R⁸, R⁹ and R¹⁰, R⁵ and R⁶, and R⁷ and R¹¹, two of the groups included in each combination (wherein these groups are alkyl groups which may have a substituent) may combine to each other to form a 5- or 6-membered ring; and M⁻ represents a counter ion; provided that combination of R¹, R², and R⁸
(1) imparts a substantially high electron density to the benzene ring to which they bond so that the compound represented by the formula (I) can be substantially non-fluorescent before trapping proton, a metal ion, or an active oxygen species, and
(2) substantially reduces electron density of the benzene ring to which they bond so that the compound derived from the compound represented by the formula (I) after trapping proton, a metal ion, or an active oxygen species can be highly fluorescent after the trapping.

2. The fluorescent probe according to claim 1, wherein the benzene ring on which R¹, R², and R³ substitute has an oxidation potential leas than 1.20 V before trapping proton, a metal ion, or an active oxygen species, and an oxidation potential not less than 1.40 V after trapping proton, a metal ion, or an active oxygen species.

3. The fluorescent probe according to claim 1 or 2, wherein R³ is a lower alkyl group, or a lower alkoxy group.

4. The fluorescent probe according to any one of claims 1 to 3, wherein the metal ion is an alkali metal ion, calcium ion, magnesium ion, or zinc ion.

5. The fluorescent probe according to any one of claims 1 to 3, wherein the active oxygen species is selected from the group consisting of nitric oxide, hydroxyl radical, singlet oxygen, and superoxide.

6. A compound represented by the following formula (II): 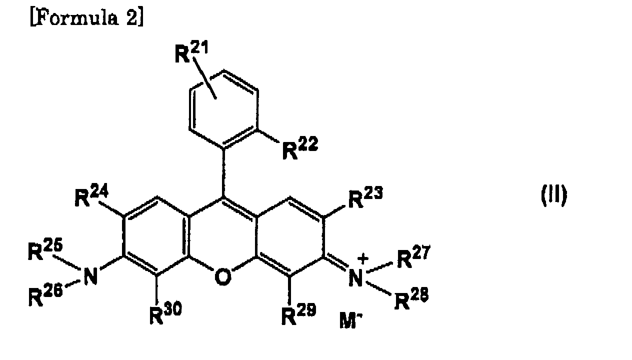 wherein R²¹ represents hydrogen atom, an alkyl group, or an alkoxy group; R²² represents an alkyl group, or an alkoxy group; R²³ and R²⁴ each independently represents hydrogen atom, a halogen atom, or an alkyl group which may have a substituent; R²⁵, R²⁸, R²⁷, and R²⁸ each independently represent an alkyl group which may have a substituent; R²⁹ and R³⁰ each independently represent hydrogen atom, a halogen atom, or an alkyl group which may have a substituent; in one or more combinations selected from the group consisting of combinations of R²⁸ and R²⁷, R²⁸ and R²⁹, R²⁴ and R²⁵, and R²⁶ and R³⁰, two of the groups included in each combination (wherein these groups are alkyl groups which may have a substituent) may combine to each other to form a 5- or 6-membered ring; and M- represents a counter ion.
